Europäisches Patentamt

(19) European Patent Office

Office européen des brevets

(11) Publication number: **0 328 420**
**A2**

# (12) EUROPEAN PATENT APPLICATION

(21) Application number: 89301347.4

(22) Date of filing: 13.02.89

(51) Int. Cl.⁴: **C 07 H 15/10**
**A 61 K 31/70, C 12 N 5/00**

(30) Priority: 12.02.88 US 155401   30.01.89 US 303211

(43) Date of publication of application:
16.08.89 Bulletin 89/33

(84) Designated Contracting States:
AT BE CH DE ES FR GB GR IT LI LU NL SE

(71) Applicant: The Biomembrane Institute
201 Elliott Avenue West Suite 305
Seattle Washington 98119 (US)

(72) Inventor: Nores, Gustavo The Biomembrane Institute
201 Elliott Avenue West
Seattle, WA 98119 (US)

Hanai, Nobuo The Biomembrane Institute
201 Elliott Avenue West
Seattle, WA 98119 (US)

Dohi, Taeko The Biomembrane Institute
201 Elliott Avenue West
Seattle, WA 98119 (US)

Nojiri, Hisao The Biomembrane Institute
201 Elliott Avenue West
Seattle, WA 98119 (US)

Hakomori, Sen-itiroh The Biomembrane Institute
201 Elliot Avenue West
Seattle, WA 98119 (US)

(74) Representative: Woodcraft, David Charles et al
BROOKES & MARTIN High Holborn House 52/54 High
Holborn
London, WC1V 6SE (GB)

(54) Gangliosides containing de-N-acetyl-sialic acid and their applications as modifiers of cell physiology.

(57) Substantially pure gangliosides containing de-N-acetyl-sialic acid. A culture medium for stimulating growth of human and animal cells comprising: (1) essential nutrients for cell growth, and (2) a cell growth stimulatory amount of one or more gangliosides containing de-N-acetyl-sialic acid. A method for stimulating growth of human and animal cells cultured in vitro comprising contacting said cells in culture with a cell growth stimulatory amount of one or more gangliosides containing de-N-acetyl-sialic acid. A medicament for stimulating growth of human and animal cells comprising: (1) a cell growth stimulatory amount of one or more growth stimulators selected from the group consisting of gangliosides containing de-N-acetyl-sialic acid, substances that promote synthesis of gangliosides containing de-N-acetyl-sialic acid and pharmaceutically acceptable salts thereof; and (2) a pharmaceutically acceptable carrier, diluent or excipient. A method for stimulating growth of human and animal cells in vivo comprising contacting said cells with a cell growth stimulatory amount of gangliosides containing de-N-acetyl-sialic acid, substances that promote synthesis of gangliosides containing de-N-acetyl-sialic acid, and pharmaceutically acceptable salts thereof. A medicament for inhibiting growth of human and animal cells comprising: (1) a cell growth inhibitory amount of one or more growth inhibitors that block synthesis of gangliosides containing de-N-acetyl-sialic acid or pharmaceutically acceptable salts thereof; and (2) a pharmaceutically acceptable carrier, diluent or excipient. A method for inhibiting growth of human and animal cells in vivo comprising contacting said cells with a cell growth inhibitory amount of one or more growth inhibitors that block synthesis of gangliosides containing de-N-acetyl-sialic acid or pharmaceutically acceptable salts thereof.

**Description**

## GANGLIOSIDES CONTAINING DE-N-ACETYL-SIALIC ACID AND THEIR APPLICATIONS AS MODIFIERS OF CELL PHYSIOLOGY

This invention relates to gangliosides containing de-N-acetyl-sialic acid (neuraminic acid) or N-trifluoroacetyl-sialic acid and their heretofore unknown effects on cell growth modification. More particularly, this invention relates to: (1) the natural occurance of gangliosides containing de-N-acetyl sialic acid in various tumor cell lines and actively growing cells; (2) the effect of these gangliosides on cell growth both in vivo and in culture medium containing these gangliosides; and (3) modification of the amino group of neuraminic acids in gangliosides which may further modify cell growth such as inhibiting tumor cell growth.

Gangliosides are one class of glycospingolipids containing a strong acid component called sialic acid (N-acyl or O-acyl neuraminic acid). Besides gangliosides three series of neutral glycolipids are known, i.e., lacto, globo, and ganglio series. In addition, the simplest glycospingolipids, containing only one carbohydrate, are known as cerebrosides. Currently, approximately 130 molecular species of glycosphingolipids are known, many of which are present at the cell surface membrane (Hakomori, S. (1986) Scientific American 254, 44-53).

Cell surface gangliosides may have two basic cellular functions: i) to mediate cell social functions (cell-cell, cell-microbe, or cell-matrix interactions), and ii) to modulate functional membrane proteins such as receptors and transporters (Hakomori, S. (1981) Ann. Rev. Biochem. 50, 733-764). Ganglioside-mediated modulation of membrane receptor function has been suggested by the modification of protein kinase activity of epidermal growth factor (EGF) and platelet derived growth factor (PDGF) receptors by specific gangliosides (GM3 or GM1), but not by other types of glycolipids (Bremer, E., et al (1984) J. Biol. Chem. 259, 6818-6825 and Bremer, E., et al (1986) J. Biol. Chem. 261, 2434-2440). For example, in A431 cells, which are characterized by a high content of EGF receptor, tyrosine phosphorylation of EGF receptors was specifically inhibited by exogenous addition of GM3 but not by other gangliosides or neutral glycolipids (Bremer, E., et al (1986) J. Biol. Chem. 261, 2434-2440). Inhibition of the receptor kinase was demonstrated on the isolated EGF receptor after adsorption on an anti-receptor-antibody-Sepharose complex (Bremer, E., et al (1986) J. Biol. Chem. 261, 2434-2440). Further, exogenous addition of these gangliosides, GM3 and GM1, affects fibroblast growth factor- (FGF), EGF-, or PDGF-dependent cell growth stimulation in chemically-defined media (Bremer, E. et al (1986) J. Biol. Chem. 261, 2434-2440; Bremer, E., and Hakomori, S. (1982) Biochem. Biophys. Res. Commun. 106, 711-718; and Hakomori, S., et al (1986) in Neuronal plasticity and gangliosides (Tettamanti, G., Ledeen, R., Nagai, Y., Sandhoff, K., and Toffano, G., eds.), pp. 201-214. Liviana Press, Padova, Italy). More recently, cytoskeletal protein kinase (Tsuji, S., et al (1983) J. Biochem. (Tokyo) 94, 303-306) has been found to be modified by gangliosides and various cell growth modulators such as retinoids, butyrate and 12-0-tetradecanoylphorbol-13-acetate (TPA) have been reported to induce changes in ganglioside synthesis when cell growth is arrested (Patt, L., et al (1978) Nature 273, 379-381; Fishman, P.H., et al (1974) Biochem. Biophys. Res. Commun. 59, 292-299; Huberman, E., et al (1979) Cancer Res. 39, 2618-2624; and Burczak, J.D. et al, (1983) Exp. Cell Res. 147, 281-286). On the other hand, sphingosines have been shown to have a non-specific common inhibitory effect on protein kinase C activity (Hannun, Y.A., et al (1986) J. Biol. Chem. 261, 12604-12609).

Because of the remarkable cell growth modifying activities of gangliosides, potential application of gangliosides to modulation of in vitro and in vivo cell growth is an exciting area for investigation.

Further, because gangliosides occur naturally in many cells, use of inhibitors or promoters of synthesis of particular gangliosides to abolish or enhance the natural effects of the gangliosides is also an exciting area for investigation.

Unfortunately, because of the unpredictable cell growth modifying activities of different gangliosides which have been studied, e.g. GM3 and GM1, and because of lack of any coherent information as to what types of cells react to each ganglioside, little progress has been made in the area of in vitro or in vivo applications of gangliosides to modulate cell growth.

Accordingly, it would be desirable to be able to identify gangliosides that have the same effects on a variety of cells so that concrete in vitro or in vivo applications of the gangliosides can be identified and used for practical purposes.

An aspect of the present invention is the provision of structural information about a new type of ganglioside that contains de-N-acetyl sialic acid which has not been previously known, obtained in purified form or synthetically synthesized. A further aspect of the present invention is the in vitro application of these gangliosides to a wide range of cell types.

Yet a further aspect of the present invention is the provision of evidence that these gangliosides stimulate cell growth through promotion of phosphorylation of EGF, PDGF and/or FGF receptor or insulin receptor. Accordingly, inhibitors of synthesis of gangliosides containing N-modified sialic acid, such as gangliosides that contain N-trifluoroacetyl sialic acid, in general could be used as inhibitors of growth of specific types of cells such as tumor cells.

According to the invention there is provided a substantially pure ganglioside containing de-N-acetyl-sialic acid.

The present invention also provides a culture medium for stimulating growth of human and animal cells comprising: (1) essential nutrients for cell growth, and (2) cell growth stimulatory amounts of one or more gangliosides containing de-N-acetyl-sialic acid.

In a particular embodiment, the culture medium is for stimulating insulin-dependent growth of human and animal cells comprising: (1) essential nutrients for cell growth, and (2) a cell growth stimulatory amount of one or more gangliosides containing de-N-acetyl-sialic acid.

The present invention further provides a method for stimulating growth of human and animal cells cultured in vitro comprising contacting the cells in culture with a cell growth stimulatory amount of one or more gangliosides containing de-N-acetyl-sialic acid.

In a particular embodiment, the method is for stimulating insulin-dependent growth of human and animal cells cultured in vitro comprising contacting the cells in culture with a cell growth stimulatory amount of one or more gangliosides containing de-N-acetyl-sialic acid.

In a further aspect, the present invention provides a medicament for stimulating growth of human and animal cells comprising: (1) a cell growth stimulatory amount of one or more growth stimulators selected from the group consisting of gangliosides containing de-N-acetyl-sialic acid, substances that promote synthesis of gangliosides containing de-N-acetyl sialic acid and pharmaceutically acceptable salts thereof; and (2) a pharmaceutically acceptable carrier, diluent or excipient.

The present invention also provides a method for stimulating growth of human and animal cells in vivo comprising contacting the cells with a cell growth stimulatory amount of gangliosides containing de-N-acetyl-sialic acid, substances that promote synthesis of gangliosides containing de-N-acetyl-sialic acid and pharmaceutically acceptable salts thereof.

In preferred embodiments, this medicament and method are used to promote wound healing.

In a particular embodiment, the present invention provides a medicament for stimulating insulin-dependent growth of human and animal cells comprising: (1) a cell growth stimulatory amount of one or more gangliosides containing de-N-acetyl-sialic acid, or pharmaceutically acceptable salts thereof; and (2) a pharmaceutically acceptable carrier, diluent or excipient.

In this particular embodiment, the present invention also provides a method for stimulating insulin-dependent growth of human and animal cells in vivo comprising contacting said cells with a cell growth stimulatory amount of one or more gangliosides containing de-N-acetyl-sialic acid or pharmaceutically acceptable salts thereof.

In an even further aspect, the present invention provides a medicament for inhibiting growth of human and animal cells comprising: (1) a cell growth inhibitory amount of one or more growth inhibitors that block synthesis of gangliosides containing de-N-acetyl-sialic acid and pharmaceutically acceptable salts thereof; and (2) a pharmaceutically acceptable carrier, diluent or excipient.

The present invention also provides a method for inhibiting growth of human and animal cells in vivo comprising contacting the cells with a cell growth inhibitory amount of one or more growth inhibitors that block synthesis of gangliosides containing de-N-acetyl-sialic acid and pharmaceutically acceptable salts thereof.

In preferred embodiments, this medicament and method are used to prevent or slow tumor growth and to prevent or slow metastasis of tumors.

Further because N-trifluoroacetyl sialic acid or any other N-modified sialic acid could inhibit formation of de-N-acetyl-sialic acid, those gangliosides containing N-trifluoroacetyl sialic acid or any other N-modified sialic acid-containing gangliosides are expected to be strong inhibitors of cell growth, including tumor growth.

Also, in preferred embodiments for stimulating cell growth, the gangliosides containing de-N-acetyl-sialic acid comprise de-N-acetyl-GM3 and de-N-acetyl-GM1.

In embodiments relating to stimulating insulin-dependent cell growth, preferred gangliosides containing de-N-acetyl-sialic acid comprise sialyl 2→3 lactoneotetraosylceramide or sialyl 2→3 lactonorhexaosylceramide containing de-N-acetyl-sialic acid.

The invention will be illustrated by reference to the drawings of which;

Figure 1 shows the structures of GM3 and de-N-acetyl-GM3. The glycolipids are abbreviated according to the system of Svennerholm (Svennerholm, L. (1963) J. Neurochem. 10, 613-623) for ganglio-series gangliosides.

Figure 2 is a diagram of the reaction scheme for synthesis of de-N-acetyl-GM3.

Figure 3 is a high-performance thin-layer chromatography (HPTLC) pattern of GM3 and de-N-acetyl-GM3 (D1) using chloroform-methanol-aqueous 0.2% CaCl2 (5:4:1) and detection with resorcinol.

Figure 4 is a proton labeling scheme for NMR analysis of GM3.

Figure 5 shows resolution enhanced $^1$H-NMR spectra: (A) GM3, (B) de-N-acetyl-GM3 (D1). The resonance assignments given in (A) follow those of Koerner et al (T.A.W. Koerner, Jr., et al (1983) Biochemistry 22, 2676-2690). The peaks a-c are due to HOD, Me2SO, and acetone, respectively.

Figure 6 is a negative ion f.a.b.-mass spectra of dog erythrocyte GM3 in A: triethanolamine (TEA) matrix, B:glycerol matrix. Ions are designated as in Table I: a: triethanolamine matrix cluster ions of formula (n[TEA]-1)⁻, b: glycerol matrix cluster ions of formula (nG-1)⁻, wherein G represents glycerol.

Figure 7 is a negative ion f.a.b.-mass spectrum of de-N-acetyl-GM3 (D1) in a triethanolamine matrix. Inset: molecular ion region of spectrum in a glycerol matrix.

Figure 8 is a graph showing the reactivity of DH5 monoclonal antibody with various glycolipids determined by solid-phase radioimmunoassay. De-N-acetyl-GM3 (closed circles); GM3 (open upside down triangles); GM1 containing neuraminic acid (open squares, indicated as de-N-acetyl-GM1 in the figure); GM1 (open circles); neuraminylparagloboside (closed squares); sialylparagloboside (closed triangles).

Figure 9 shows a separation pattern of the de-N-acetyl-GM3 fraction, its thin-layer chromatography (TLC) immunostaining, and its detection as N-[$^{14}$C]-acetyl-GM3 from A431 cells. Panel A: orcinol/$H_2SO_4$ staining. Lanes 1-10, separation pattern of standard GM3 and de-N-acetyl-GM3 on HPTLC (chloroform:methanol:0.02% $CaCl_2$-$2H_2O$, 50:40:10) through high-performance liquid chromatography (HPLC) by latrobeads 6RS8010. De-N-acetyl-GM3 fractions are indicated by an asterisk at the bottom. Lane a, standard GM1, GM2, and GM3; lane b, standard de-N-acetyl-GM3 (upper band). Panel B: immunostaining pattern of HPTLC plate, using monoclonal IgG3 antibody DH5 directed to de-N-acetyl-GM3 and its lactone. Lanes 1-10, the separated monoganglioside fractions from A431 cell extracts through HPLC under the same conditions as shown in panel A; lane c, standard de-N-acetyl-GM3; lane d, standard GM1, GM2, GM3 - Positively stained de-N-acetyl-GM3 bands are indicated by arrows. Panel C: the N-[$^{14}$C]-acetylated de-N-acetyl-GM3 fraction from A431 cells separated on HPTLC (chloroform:methanol:0.02% $CaCl_2$-$2H_2O$, 50:40:10), and followed by autoradiography. The position of N-[$^{14}$C]-acetylated GM3 is indicated by the arrows. Lane 1, N-[$^{14}$C]-acetylated standard de-N-acetyl-GM3; lane 2, N-[$^{14}$C]-acetylated de-N-acetyl-GM3 fraction from A431 cells.

Figure 10 shows de-N-acetyl-GM3 detection in the monosialoganglioside fraction of various cell lines and tissues by TLC immunostaining with monoclonal antibody DH5. Panel A: resorcinol/HCl staining; panel B: immunostaining. Lane 1, standard GM3, GM1, de-N-acetyl-GM3, de-N-acetyl-GM1 (GM1 containing neuraminic acid); lane 2, B16 melanoma tumor grown in vivo; lane 3, B16 cells cultured in vitro; lane 4, Swiss 3T3 cells cultured in vitro; lane 5, rat liver; lane 6, rat brain.

Figure 11 is a graph showing EGF receptor kinase activity of A431 cell membranes: dependence on Triton X-100 concentration and on GM3 and de-N-acetyl-GM3. Open circles, control without ganglioside addition; closed triangles, with 500 μM GM3; closed circles, with 250 μM de-N-acetyl-GM3.

Figure 12 is two graphs showing the effect of de-N-acetyl-GM3 on in vitro phosphorylation of EGF receptor depending on ganglioside concentration (A) and on EGF concentration (B). Concentration of Triton X-100 was 0.025%. Panel A: EGF concentration was 0.33 μM. Panel B: de-N-acetyl-GM3 concentration was 500 μM. Closed circles de-N-acetyl-GM3; open circles, control without ganglioside addition; (X), no EGF.

Figure 13 is an autoradiograph of two-dimensional thin-layer electrophoresis of amino acid phosphates from a hydrolysate of EGF receptor band excised from a gel region containing EGF receptor. Panel A: only EGF; panel B: EGF and de-N-acetyl-GM3. Ser: serine phosphate, Thr: threonine phosphate, Tyr: tyrosine phosphate.

Figure 14 is graphs showing the effect of exogenously added de-N-acetyl-GM3 and GM3 on cell growth. A431 cells (panel A), Swiss 3T3 cells (panel B), and B16 cells (panel C). On the 3rd day of culture, 10 μM de-N-acetyl-GM3 (closed circles) was added to A431 cells (panel A). Fifty μM de-N-acetyl-GM3 (closed circles) was added to Swiss 3T3 cells (panel B). Fifty μM de-N-acetyl-GM3 (closed circles) or 50 μM GM3 (closed triangles) was added to B16 cells (panel C). For control (open circles) no ganglioside was added. Each data point shown is the average of 4 determinations ± S.E.

## DETAILED DESCRIPTION OF THE INVENTION

For the purposes of this invention, the following terms have the following meanings:

Ganglioside -- Glycospingolipids containing sialic acid (nonulosaminic acid or neuraminic acid containing an N-acetyl, N-glycolyl or O-acetyl group). The nomenclature used to designate ganglio-series gangliosides (e.g. GM1, GM2, GM3, etc.) is according to Svennerholm, L. (1963) J. Neurochem. 10, 613-623.

Lyso form ganglioside -- glycospingolipids in which N-fatty acyl groups linked to the amino group of spingosine are eliminated.

Ganglioside containing de-N-acetyl-sialic acid --A ganglioside, as defined above, that contains neuraminic acid. The term "neuraminic acid" is used as originally defined by Blix, Gottschalk, and Klenk (Nature 179, 1088 (1957)), i.e. nonulosaminic acid without N- or O- substitution.

De-N-acetyl-GM3 -- the structure of GM3 ganglioside containing neuraminic acid, i.e. de-N-acetyl sialic acid.

De-N-acetyl-GM1 -- the structure of GM1 ganglioside containing neuraminic acid.

Sialyl 2→3 lactonorhexaosylceramide -- a ganglioside having the structure:$VI^3$NeuAcnLc6.

Sialyl 2→3 lactoneotetraosylceramide -- a ganglioside having the structure: $IV^3$NeuAcnLc4.

Of the gangliosides containing de-N-acetyl-sialic acid useful in the present invention, one, de-N-acetyl-GM3 has been shown by the present inventors to exist in nature as a component of cells. This is the first time that any ganglioside containing de-N-acetyl-sialic acid has been identified, either as naturally existing or as a synthetic compound, and shown to display biological activities.

These gangliosides were found in various tumor cell lines and human tumor tissue but have not been detected in normal cell lines and normal human tissue.

According to the present invention it has been unexpectedly found that gangliosides containing de-N-acetyl-sialic acid possess a strong stimulatory effect on growth of numerous human and animal cell lines when added to culture media. These modified gangliosides also promote growth factor receptor associated kinase activities. Additionally, some gangliosides containing de-N-acetyl-sialic acid promote insulin dependent cell growth.

However, the particular growth factor receptor associated kinase affected appears to differ depending upon the particular ganglioside that is modified, e.g. GM3, GM1, sialyl 2→3 lactonorhexaosylceramide or sialyl 2→3 lactoneotetraosylceramide.

For example, de-N-acetyl-GM3 gangliosides appear to exert their effects by acting on EGF and PDGF receptor kinase, whereas de-N-acetyl-GM1 gangliosides appear to exert their effects by acting on PDGF receptor kinase. In contrast, de-N-acetyl 2→3 lactonorhexaosylceramide and de-N-acetyl 2→3 lactoneotetraosylceramide are expected to exert their effects by acting on insulin receptor kinase. However, the precise manner in which the modified gangliosides actually act, e.g. on various kinases, is not firmly established and, accordingly, the inventors do not want to be bound by the above explanation.

As a result of the discovery by the present inventors of the growth stimulatory effects of gangliosides containing de-N-acetyl-sialic acid, the present invention provides a culture medium for stimulating growth of human and animal cells comprising: (1) essential nutrients for cell growth, and (2) a cell growth stimulatory amount of one or more gangliosides containing de-N-acetyl-sialic acid.

The present invention also provides a method for stimulating growth of human and animal cells cultured in vitro comprising contacting said cells in culture with a cell growth stimulatory amount of one or more gangliosides containing de-N-acetyl-sialic acid.

Examples of suitable gangliosides containing de-N-acetyl-sialic acid which can be used in the culture medium and method include de-N-acetyl-GM3, de-N-acetyl-GM1, de-N-acetyl GM2, and GD1a, GD1b, GT1b, containing de-N-acetyl-sialic acid, etc.

De-N-acetyl-GM3 and de-N-acetyl-GM1 are preferred and de-N-acetyl-GM3 is especially preferred.

For insulin-dependent cell growth, sialyl 2→3 lactoneotetraosylceramide and sialyl 2→3 lactonorhexaosylceramide containing de-N-acetyl-sialic acid are preferred, and sialyl 2→3 lactoneotetraosylceramide containing de-N-acetyl-sialic acid is especially preferred.

The de-N-acetyl-gangliosides can be synthesized as described below in detail.

As the culture media that can be used in the present invention, any culture medium conventionally used to culture the particular human or animal cells of interest can be used. Such a culture medium would, at a minimum include essential nutrients for cell growth, i.e., glucose, amino acids, glutamine, vitamins, insulin, transferrin, hydrocortesone, trace metal elements, and, depending on the cells, EGF, PDGF, and/or FGF.

Enriched media can also be used.

Further, the media can be liquid or semi-solid, (e.g. including soft agar or methylcellulose gel).

Examples of suitable culture media include Eagle's Basic Medium, Dulbecco's Modified Eagle's Medium, Ham's Medium, Sato's Chemically Defined Medium, etc.

The gangliosides containing de-N-acetyl-sialic acid are added to the cell culture medium as follows.

Purified ganglioside containing de-N-acetyl-sialic acid is dissolved in a suitable organic solvent (e.g. chloroform:methanol (2:1 v/v) and evaporated under an inert atmosphere (e.g. a nitrogen stream). The residue is then dissolved in the culture medium at the desired concentration, sonicated, and passed through a sterilizing filter (e.g. 0.2 $\mu$ pore diameter).

The media can be prepared fresh for use or can be prepared and then stored for as long as one month or more at 4°C.

The gangliosides containing de-N-acetyl-sialic acid are added to the final culture media in a concentration sufficient to promote cell growth. Whether a particular cell line is susceptible to the growth promoting effect of the gangliosides containing de-N-acetyl-sialic acid and at what concentrations susceptability is exhibited can readily be determined by the skilled artisan by culturing cells in a series of concentrations of the gangliosides containing de-N-acetyl-sialic acid and then determining the growth of the cells over time at each concentration point as compared to a standard where no modified ganglioside is added.

In the case of tumor cells, which synthesize gangliosides containing de-N-acetyl-sialic acid, the growth promoting effects are small. However, normal cells which do not appear to synthesize gangliosides containing de-N-acetyl-sialic acid react strongly to exogenous addition of gangliosides containing de-N-acetyl-sialic acid.

Suitable concentrations of gangliosides containing de-N-acetyl-sialic acid that stimulate cell growth generally range from about 10 to about 30 $\mu$g/ml.

The above-described culture media and method for stimulating cell growth of human and animal cells are espcially applicable to mammalian cells such as, for example, 3T3 cells, WI38 cells, BKH cells, etc.

Cell lines that appear less susceptible to the growth promoting effects of the exogenously added de-N-acetyl-GM3 or de-N-acetyl-GM1 are those cells which are capable of producing those gangliosides endogenously, such as B16 myeloma cells.

By use of the above described culture media and method, human and animal cell growth in vitro can be increased by as much as 50-100% or more.

As mentioned above, the inventors have detected the natural occurrence of gangliosides containing de-N-acetyl-sialic acid in human and animal cells, and thus not only can these gangliosides be used to stimulate cell growth, but additionally, substances that promote or block the cellular synthesis of these modified gangliosides can also be used in the present invention to inhibit or stimulate, respectively, growth of human and animal cells that synthesize these modified gangliosides.

The natural occurrence of the modified gangliosides in various cells can be detected by the following method.

Cells are cultured according to methods appropriate for the cells, harvested, and pelleted by centrifugation.

The pellets are extracted with a suitable solvent (e.g. chloroform:methanol (2:1, v/v/)), followed by three partitions with water according to known methods, such as the modified method of Folch (Folch-Pi, J., et al (1951) J. Biol. Chem. 191, 819-831). The Folch upper phases are then combined, evaporated to a convenient volume, and salt is removed by suitable means, such as by passing the sample through a C18 silica gel column (Kundu, S.K. and Sukuzi, A. (1985) J. Chromatogr. 224, 249-256), followed by DEAE-Sephadex Chromatography (Ledeen, R.W. and Yu, R.K. (1982) Math. Enzymol. 83, 139-191 and Nores, G. and Caputto, R. (1984) J. Neurochem. 42, 1205-1211). The monosialoganglioside fraction is then eluted with a suitable solvent (e.g. chloroform:methanol:0.08 M ammonium formate (30:60:8, v/v/v), desalted again (e.g. with a C18 silica gel column) and evaporated to dryness (e.g. in a rotary evaporator).

The dried material is then subjected to a method for fractionating the particular gangliosides.

For example, the dried material can be applied to a porous silica gel column of Iatrobeads 6RS8010 (a porous silica gel manufactured by Iatron Chemical Co., Kanda, Tokyo, Japan) which is equilibrated with n-propanol-15% aqueous ammonium hydroxide (75:15, v/v), and eluted with a gradient from the same solvent to n-propanol-15% aqueous ammonium hydroxide (75:25 v/v) over an amount of time sufficient to elute the desired gangliosides in separate fractions (e.g. over 200 minutes using Varian 500 HPLC equipment). The eluates are collected in fractions suitable to keep the desired gangliosides separate (e.g. 2 ml/fraction when eluting over 200 minutes using Varian 500 HPLC equipment), and each fraction is analyzed by HPTLC.

By the above method, unmodified gangliosides and gangliosides containing de-N-acetyl-sialic acid can be eluted separately in that order and with no overlap.

The particular gangliosides eluted can be identified by methods described below (e.g. NMR spectroscopy and negative ion f.a.b.).

According to this method, it is possible to determine whether particular cells carry gangliosides containing de-N-acetyl-sialic acid and thus whether treatment with stimulators and/or inhibitors of synthesis of these modified gangliosides would be appropriate.

As a result of the discovery by the present inventors of the growth stimulatory effects of gangliosides containing de-N-acetyl-sialic acid and the presence of de-N-acetyl-gangliosides in various cells, the present invention provides a medicament for stimulating growth of human and animal cells comprising:

(1) a cell growth stimulatory amount of one or more growth stimulators selected from the group consisting of gangliosides containing de-N-acetyl-sialic acid, substances that promote synthesis of gangliosides containing de-N-acetyl-sialic acid and pharmaceutically acceptable salts thereof, and

(2) a pharmaceutically acceptable carrier, diluent or excipient.

Similarly, the present invention also provides a method for stimulating growth of human and animal cells in vivo comprising contacting said cells with a cell growth stimulatory amount of gangliosides containing de-N-acetyl-sialic acid, substances that promote synthesis of gangliosides containing de-N-acetyl-sialic acid, and pharmaceutically acceptable salts thereof.

As the ganglioside containing de-N-acetyl-sialic acid or its pharmaceutically acceptable salts, any ganglioside containing de-N-acetyl-sialic acid as defined above or its pharmaceutically acceptable salt can be used.

Examples of suitable gangliosides containing de-N-acetyl-sialic acid which can be used in the medicament and method for stimulating cell growth in vivo include those described above for use in the culture medium of the present invention and their pharmaceutically acceptable salts.

De-N-acetyl-GM$_3$ and de-N-acetyl-GM$_1$ are preferred and de-N-acetyl-GM$_3$ is especially preferred.

Suitable substances and their pharmaceutically acceptable salts that promote synthesis of gangliosides containing de-N-acetyl-sialic acid can be determined by the skilled artisan and include, for example, N-acetylase.

N-acetylase can be obtained by methods readily determined by the skilled artisan.

The above-described medicament and method for stimulating in vivo growth of human and animal cells are especially applicable to treatment of mammalian cells and especially to cells that are involved in wound healing.

In a more particular embodiment, the present invention provides a medicament for stimulating insulin-dependent growth of human and animal cells comprising: (1) a cell growth stimulatory amount of one or more gangliosides containing de-N-acetyl-sialic acid, or pharmaceutically acceptable salts thereof; and (2) a pharmaceutically acceptable carrier, diluent or excipient.

The present invention, in this more particular embodiment, also provides a method for stimulating insulin-dependent growth of human and animal cells in vivo comprising contacting said cells with a cell growth stimulatory amount of one or more gangliosides containing de-N-acetyl-sialic acid or pharmaceutically acceptable salts thereof.

Examples of suitable gangliosides containing de-N-acetyl-sialic acid or their pharmaceutically acceptable salts include those defined above for use in the culture medium for stimulating cell growth and their pharmaceutically acceptable salts.

Sialyl 2→3 lactoneotetraosylceramide and sialyl 2→3 lactonorhexaosylceramide containing de-N-acetyl-sialic acid are preferred, and sialyl 2→3 lactoneotetraosylceramide containing de-N-acetyl-sialic acid is especially preferred.

Also as a result of the discovery by the present inventors of the growth stimulatory effect of gangliosides containing de-N-acetyl-sialic acid in various cells, the present invention provides a medicament for inhibiting growth of human and animal cells comprising:

(1) a cell growth inhibitory amount of one or more growth inhibitors that block synthesis of gangliosides containing de-N-acetyl-sialic acid and pharmaceutically acceptable salts thereof, and

(2) a pharmaceutically acceptable carrier, diluent or excipient.

Similarly, the present invention also provides a method for inhibiting growth of human and animal cells in vivo comprising contacting said cells with a cell growth inhibitory amount of one or more growth inhibitors that block synthesis of de-N-acetyl-gangliosides or pharmaceutically acceptable salts thereof.

Suitable substances and their pharmaceutically acceptable salts that block synthesis of gangliosides containing de-N-acetyl-sialic acid can be determined by the skilled artisan, and include, for example, anti-N-deacetylase. Various N-modified gangliosides and lyso form gangliosides such as gangliosides and lyso form gangliosides containing N-trifluoroacetyl sialic acid, N-carbamyl acetyl sialic acid, N-propyl sialic acid, N-dichloroacetyl sialic acid and N-trichloroacetyl sialic acid are also considered appropriate inhibitors of synthesis of de-N-acetyl gangliosides.

Anti-N-deacetylase can be obtained by methods readily determined by the skilled artisan as can the various N-modified gangliosides and N-modified lyso form gangliosides (Hakomori, S. et al. (1980) "Cell Biological and Immunological Significance of Ganglioside Changes Associated with Transformation" in Structure and Function of Gangliosides, (Svennerholm, L. Dreyfus,H., Urban, P.S. - eds) Plenum Publishing Corp., N.Y. pp 247-261).

The above described medicament and method for inhibiting growth of human and animal cells are especially applicable to treatment of mammalian cells and especially to malignant or non-malignant tumor cells to prevent or slow tumor growth and to prevent or slow metastasis of tumors.

Suitable pharmaceutically acceptable carriers, diluents or excipients for the medicaments of the present invention depend upon the particular medical use of the medicament and can readily be determined by the skilled artisan.

Suitable methods of administration of the medicaments of the present invention depend upon the particular medical application and can readily be determined by the skilled artisan.

Suitable doses of the medicaments of the present invention depend upon the particular medical application, as well as the weight and sex of the subject, etc., and can readily be determined by the skilled artisan from in vitro data.

The synthesis of the novel gangliosides containing de-N-acetyl-sialic acid of the present invention will now be described.

The synthesis of the modified ganglioside can be accomplished by using the corresponding unmodified ganglioside containing N-acetyl neuraminic acid as the starting material.

The unmodified ganglioside containing N-acetyl neuraminic acid to be used as the precursor for the ganglioside containing de-N-acetyl-sialic acid is obtained by extraction from an appropriate source readily known to the skilled artisan (e.g. dog erythrocytes for $GM_3$ and bovine brain for most other gangliosides) and purified by chromatography (R.K. Yu and R.W. Ledeen (1972) J. Lipid Res. 13, 680-686) on DEAE Sephadex followed by high-performance liquid chromatography on a column of Iatrobeads 6RS8010 using various gradient elution systems well known in the art (for example, see K. Watanabe and Y. Arao (1981) J. Lipid Res. 22, 1020-1024).

The thus isolated unmodified ganglioside containing N-acetyl neuraminic acid is then used to prepare the ganglioside containing de-N-acetyl-sialic acid. A critical step in the preparation of the ganglioside containing de-N-acetyl-sialic acid is the use of mild alkaline conditions of hydrolysis under which the N-acetyl group of sialic acid is preferentially hydrolyzed.

Specifically, the N-acetyl group of the sialic acid portion of the unmodified ganglioside is preferentially hydrolyzed to give the de-N-acetyl form on treatment with 0.1 M NaOH in aqueous 90% 1-butanol at about 80° for about 4 hours. The yield is generally greater than 70%. The hydrolysate in aqueous 1-butanol is then neutralized with acid, e.g. 12 M HCl, and taken to dryness. The residue is resuspended in water in any convenient volume and passed through a column of $C_{18}$ silica. The column is washed with water to eliminate salts and then the lipids, mostly gangliosides containing de-N-acetyl-sialic acid, are eluted with an appropriate solvent (e.g., methanol). The derivative is purified by high-performance liquid chromatography.

The synthesis of the ganglioside containing de-N-acetyl-sialic acid will now be described by reference to the following synthesis example which is illustrated in Figure 2.

$GM_3$ ganglioside. -- $GM_3$ containing N-acetyl-neuraminic acid was extracted from dog erythrocytes and purified by chromatography (R.K. Yu and R.W. Ledeen (1972) J. Lipid Res. 13, 680-686) on DEAE Sephadex followed by high-performance liquid chromatography on a column of Iatrobeads 6RS8010 in a 2-propanol-hexane-water system (K. Watanabe and Y. Arao (1981) J. Lipid Res., 22, 1020-1024). The behavior on high performance thin-layer chromatography is shown in Figure 3.

Preparation of de-N-acetyl-$GM_3(D_1)$ -- On hydrolysis with 0.1 M NaOH in aqueous 90% 1-butanol at 80° for 4 hours, the N-acetyl group of $GM_3$ sialic acid was preferentially hydrolyzed to give de-N-acetyl-$GM_3$, which has a free amino group at the sialic acid moiety of $GM_3$ (See Figure 4), as the main product (>70%). The hydrolysate in aqueous 1-butanol was neutralized with 12 M HCl, concentrated to dryness, and a solution of the residue in water (6 ml) was passed through a column of $C_{18}$ silica (Bond Elut, Analytichem International, Oxnard, CA). After washing with water to eliminate salts, the lipids were diluted with methanol. The derivatives were finally purified by high-performance liquid chromatography. The behavior on high-performance thin-layer chromatography is shown in Figure 3.

The procedures described for GM3 can be applied to any ganglioside to give derivatives which are useful in the present invention.

Characterization of de-N-acetyl-GM3 --De-N-acetyl-GM3 was characterized by NMR spectroscopy and negative ion fast atom bombardment (f.a.b.) spectrometry. Solutions of the compound (400 μg) in Me2SO-d6-D2O (98:2, 0.3 ml) were stored for 5 minutes to allow deuterium exchange of hydroxy and amino protons. Each solution was then lyophilized and a solution of the residue Me2SO-d6-D2O (98:2, 0.5 ml) was used immediately for NMR spectroscopy. $^1$H-NMR spectra were obtained at 35° with a Bruker WM-500 spectrometer equipped with an Aspect 2000 computer using quadrature detection, a spectral width of 5000 Hz over 16K data points, and a relaxation delay of 2 s. The number of transients collected varied from 200 to 500. Chemical shifts are referenced to the terminal methyl resonance(s), the shift of the resonance of which was assumed to be 0.85 p.p.m.

The structure and proton labeling scheme for GM3 and de-N-acetyl-GM3 (D1) are shown in Figure 4.

Negative ion f.a.b.-mass spectrometry was performed using a JEOL HX-110 mass spectrometer/DA-5000 data system. Solutions of samples in methanol were transferred to a triethanolamine or glycerol matrix on the f.a.b. target and bombarded with a xenon beam. The acceleration voltage was 10 kV and the resolution was 3000. Data were acquired in the accumulation mode from 100-1500 a.m.u. (atomic mass units) with a scan slope of 1 min/decade. Each spectrum represents the accumulation of three scans. Sodium iodide in glycerol was used as the mass calibration standard.

Figures 5A and 5B show the $^1$H-NMR spectra of GM3 and de-N-acetyl-GM3, respectively, along with selected resonance assignments.

The spectrum of GM3 (Figure 5A) is identical to that previously reported (T.A.W. Koerner Jr., et al (1983) Biochemistry 22, 2676-2690) and the assignments follow those of these authors, except for those of the amide protons, which were not assigned (T.A.W. Koerner Jr., et al 1983) Biochemistry 22, 2676-2690). The amide assignments were made by comparison with the spectrum of de-N-acetyl-GM3, and agree with the assignments for GM3 in Me2SO-d6 (deuterated dimethyl sulfoxide) at 35° (S. Gasa et al (1983) J. Lipid Res. 24, 174-182). Of special note for the identification of de-N-acetyl-GM3 are the resonances for the NAc of the sialic acid (A-11, δ 1.89) and the amide proton (A-N, δ 7.98).

Figure 5B shows the spectrum of de-N-acetyl-GM3 which differs from that of GM3 by the absence of the resonances for the sialic acid acetamido methyl group (A-11, δ 1.89) and the sialic acid amide proton (A-N, δ 7.98). In addition, the resonance for sialic acid H-7, which appears at δ 3.1 in the spectrum of GM3, moved down-field in the spectrum of de-N-acetyl-GM3, whereas that of H-3e moved up-field (-0.1 p.p.m.). These shifts may be explained by loss of the anisotropic effect of the carbonyl of the acetamido group formerly bound to neuraminic acid.

The major fragments observed in the mass spectra of GM3 and of de-N-acetyl-GM3 (D1) obtained in both triethanolamine and glycerol matrices are summarized in Table I and the spectra are shown in Figures 6 and 7, respectively.

TABLE I

Mass ($\underline{m}/\underline{z}$) of relevant fragments produced by negative ion f.a.b.-mass spectrometry of $GM_3$ and synthetic derivatives

| | $R_1$ | $R_2$ | M-H | M-A | M-A,II | M-A,II,I |
|---|---|---|---|---|---|---|
| $GM_3$ | $COCH_3$ | $COC_{23}H_{45}$ | 1261 | 970 | 808 | 646 |
| $D_1$ | H | $COC_{23}H_{45}$ | 1219 | 970 | 808 | 646 |

The negative ion f.a.b.-mass spectrum of $GM_3$ in a triethanolamine matrix (Figure 6A) was dominated by the pseudo-molecular ions, some fragmention occurring through loss of sugar residues from the non-reducing end, with charge retention on the ceramide-containing portion of the molecule. The presence in canine erythrocyte $GM_3$ of ceramide composed almost exclusively of d18:1 sphingosine and 24:1 (+24:0) fatty acid, was indicated by the abundant ion at $\underline{m}/\underline{z}$ 1261 (1263). The fragment expected from d18:1 sphingosine, at $\underline{m}/\underline{z}$ 237, was not observed in the spectrum.

In the spectrum obtained using a glycerol matrix (Figure 6B), the same ions were observed ($\underline{m}/\underline{z}$ 1261, 970, 808, 646), along with a number of glycerol cluster ions (nG-1)⁻. The spectrum of de-N-acetyl-$\overline{GM}_3$ (D₁) in a triethanolamine matrix was characterized by the unexpected presence, in addition to the abundant pseudomolecular (M-H)⁻ ions, of ions 26 and 42 a.m.u. higher in mass.

While not wanting to be bound by their explanation, the present inventors postulate that the presence of these ions is related to the number of free amino functions in the molecule, and that they are the result of some reaction taking place during the bombardment process in a triethanolamine matrix.

The de-N-acetylation of $GM_3$ containing N-acetyl neuraminic acid to produce de-N-acetyl-$GM_3$ (D₁) should reduce the mass of the pseudomolecular ions by 42 a.m.u. In the mass spectrum of de-N-acetyl-$GM_3$ in a triethanolamine matrix (Figure 7), the expected predominant pseudomolecular ion was found at $\underline{m}/\underline{z}$ 1219 (1221) for 24:1 (+24:0) fatty acid containing ceramides, accompanied by associated (M-H+26)⁻ and (M-H+42)⁻ ions at $\underline{m}/\underline{z}$ (1247) and 1261 (1263). The mass spectrum of de-N-acetyl-$GM_3$ in a glycerol matrix (Figure 7, inset) exhibited only the pseudomolecular ions ($\underline{m}/\underline{z}$ 1219, 1221), with little useful fragmentation. The abundant fragment ions seen with the triethanolamine matrix ($\underline{m}/\underline{z}$ 970, 972, 808, 810, 646, 648) were unaccompanied by additional higher mass clusters, since the terminal neuraminic acid possessing the free amino function was eliminated in the production of these fragments. The fact that the difference between (M-H)⁻ and (M-A)⁻ is 249 a.m.u., instead of 291 a.m.u. expected for loss of N-acetyl neuraminic acid, confined the missing 42 a.m.u. to that residue, confirming its de-N-acetylation.

## EXAMPLES

The present invention will now be described by reference to specific examples, but the invention is not to be construed as being limited thereto.

Unless otherwise specified, all percents, ratios, etc., are by weight.

## EXAMPLE I

### DEMONSTRATION OF DE-N-ACETYL-GM₃ IN VARIOUS CELL LINES AND TISSUES

Preparation of glycolipids and modified glycolipids

GM₃ and de-N-acetyl-GM₃ were prepared according to the synthesis example set forth in the Detailed Description of the Invention section. GM₁ and GM₂, used as standards, were prepared by extraction from bovine brain by standard methods, followed by extracton with organic solvent, Folch's solvent partition, DEAE sephedex chromatography, HPLC, etc. as described in, for example, Sphingolipid Biochemistry, Plenum Press, S. Hakomori (1983). De-N-acetyl-GM₁ was prepared by a method analogous to that set forth in the Synthesis Example, except that the starting material was extracted from bovine brain.

Cell Culture

The A431 human vulvar epidermoid carcinoma cell line (Fabricant, R.N. et al (1977) Proc. Natl. Acad. Sci. USA 74, 565-569), Swiss 3T3 mouse fibroblast cell line, and B16 mouse melanoma cell line were used in this study. The medium used for the culture was basal medium (a mixture of DME and F-12 in the volume ratio of 1:1). Cells were grown in basal medium supplemented with 5% calf serum. Cells were also grown in serum-free media supplemented with 5 μg/ml insulin, 5 μg/ml of transferrin, 5 ng/ml sodium selenate, and 100 μg/ml fatty acid-free BSA including 1% linoleic acid (serum-free defined DME/F-12 medium). For the culture of Swiss 3T3 cells in serum-free medium, 10 ng/ml EGF and 10 ng/ml PDGF were added to serum-free defined DME/F-12 medium. To culture Swiss 3T3 cells and B16 cells in serum-free medium, plates were coated with fibronectin in advance by incubating 40 μl of 10 μg/ml fibronectin in PBS for 2 hours at room temperature.

Culture of B16 melanoma cells grown in mice as in vivo tumor

B16 melanoma cells were also prepared by growing them in syngeneric mice as in vivo tumors by methods well known in the art.

Sources of tissue samples

Normal rat brain tissue samples were obtained from laboratory rats (Wistar Laboratories, Philadelphia, PA). Normal rat liver tissue samples were obtained from laboratory rats (Wistar Laboratories, Philadelphia, PA). Human colonic cancer tissue was obtained from surgical samples.

Ganglioside extraction from cells and tissues

Cultured cells were harvested using a rubber policeman, and pelleted by centrifugation. Cells from tissues were passed through a wire mesh and also pelleted by centrifugation. The cell pellets were extracted with chloroform:methanol (2:1, v/v), followed by three partitions with $H_2O$ according to the modified method of Folch (Folch-Pi, J., et al (1951) J. Biol. Chem. 191, 819-831). The Folch upper phases were combined, evaporated to a small volume, and freed from salt with a C18 silica gel column (Kundu, S.K. an Suzuki, A. (1985) J. Chromatogr. 224, 249-256) followed by DEAE-Sephadex chromatography (Ledeen, R.W. and Yu, R.K. (1982) Meth. Enzymol. 83, 139-191 and Nores, G. and Caputto, R. (1984) J. Neurochem. 42, 1205-1211). The monosialoganglioside fraction eluted with chloroform:methanol:0.08 M ammonium formate (30:60:8, v/v/v) was desalted with a C18 silica gel column and evaporated to dryness in a rotary evaporator. The dried material was applied on a porous silica gel column of Iatrobeads 6RS8010, which was equilibrated with n-propanol-15% aqueous ammonium hydroxide (75:15, v/v), and eluted with a gradient from the same solvent to n-propanol-15% aqueous ammonium hydroxide (75:25 v/v) during 200 minutes in Varian 500 HPLC equipment. The eluates were collected over a fraction collector (2 ml/fraction), and each fraction was analyzed on HPTLC. Under these conditions, GM₃ and de-N-acetyl-GM₃ were eluted in that order and clearly separated without overlap.

N-[¹⁴C]-acetylation of gangliosides

For each cell line or tissue, the fraction containing de-N-acetyl-GM₃ was N-acetylated with [¹⁴C]-acetic anhydride in methanol containing 0.1 M NaHCO₃ as follows.

Dried samples were dissolved in 50 μl of 0.5 M NaHCO₃ by stirring and heating at 60°C. Twenty-five μCi ¹⁴C-acetic anhydride (5.1 mCi/mmol) in 0.1 ml of hexane was added and incubated for 1 hour at room temperature. After drying under a nitrogen stream, the reaction mixture was dissolved in distilled water and loaded on a $C_{18}$-silica gel column. Labeled glycolipid was eluted with chloroform:methanol (2:1, v/v) and analyzed by HPTLC (chloroform:methanol:0.02% $CaCl_2$-$2H_2O$, 60:40:9) through HPLC by latrobeads 6RS8010.

Additionally, the de-N-acetyl-GM3 fraction from half of 25 g of pelleted cells or tissue was N-[$^{14}$C]-acetylated and separated on HPTLC (chloroform:methanol:0.02% $CaCl_2$-$2H_2O$, 50:40:10), and followed by autoradiography.

## Preparation of monoclonal antibody DH5, which specifically reacts with de-N-acetyl-GM3

De-N-acetyl-GM3, prepared as described in the synthesis example in the Detailed Description of the Invention, was adsorbed on acid-treated Salmonella minnesotae. Alternatively, the compound was lactonized by being dissolved in chloroform: methanol:12 N HCl (60:30:4.5 v/v/v) and allowed to stand for 18 hours (Nores, G., et al (1987) J. Immunol. 139, 3171-3176). The hydrochloric acid was evaporated under a nitrogen stream, and the residue was dissolved in water, adsorbed on acid-treated S. minnesotae, and used as immunogen according to the method previously described (Nores, G., et al (1987) J. Immunol. 139, 3171-3176 and Young, W.W. Jr., et al (1979) J. Exp. Med. 150, 1008-1019). The immunization protocol and procedures for preparing, selecting and cloning of monoclonal antibodies were also conducted according to previously described known methods (Nores, G., et al (1987) J. Immunol. 139, 3171-3176 and Young, W.W. Jr., et al (1979) J. Exp. Med. 150, 1008-1019).

The hybridoma secreted IgG3 antibody DH5, which specifically reacts with de-N-acetyl-GM3 and neuraminyl 2→3 paragloboside (IV$^3$NeuNH2nLc4), but does not react with GM3 or any other ganglioside, including GM1 containing neuraminic acid (III$^3$NeuNH2Gg4).

Specificity of this monoclonal antibody was determined by solid-phase radioimmunoassay by a procedure previously described (Kannagi, R., et al (1983) Cancer Res. 43, 4997-5005).

Briefly, 20 pmole/well of de-N-acetyl-GM3, GM3, de-N-acetyl-GM1, GM1, neuraminylparagloboside (prepared by de-N-acetylation of sialylparagloboside which was obtained from human erythrocytes (Sphingolipid Biochemistry, Plenum Press, S. Hakomori, (1983)), or sialylparaglobside (isolated from human erythrocytes (Sphingolipid Biochemistry, Plenum Press, S. Hakamori, (1983)) with 50 ng/well phosphatidylcholine and 30 ng/well cholesterol in ethanol were dried onto 96 well plastic plates. The wells were blocked with 5% BSA in PBS for 2 hours and reacted with various concentrations (see Figure 8) of monoconal antibody DH5 for 2 hours at room temperature. Bound antibody was detected using rabbit anti-mouse IgG followed by $^{125}$I-protein A. The radioactivity of each well was counted with a gamma counter.

The results are shown in Figure 8. As can be seen from the results, the only ganglioside to which monoclonal antibody DH5 is specific is de-N-acetyl-GM3.

Analogous methods can be used to produce monoclonal antibodies specific to other gangliosides containing de-N-acetyl-sialic acid.

## TLC Immunostaining

TLC Immunostaining was carried out as described by Magnani et al (Magnani, J.L. et al (1980) Anal. Biochem. 109, 399-402).

Specifically, the monosialoganglioside fraction from a 100 mg cell or tissue pellet was spotted on TLC plates for chromatography using a solvent system of chloroform:methanol:water (50:40:10) containing 0.05% $CaCl_2$. TLC plates were coated with plastic, blocked with 5% BSA in PBS, and reacted with culture supernatant of monoclonal antibody DH5-producing hybridomas for 2 hours at room temperature. Bound antibody was detected using a rabbit anti-mouse IgG antibody followed by $^{125}$I-protein A, and tested by autoradiography.

The results are shown in Figures 9 and 10.

Figure 9, panel A shows the separation pattern (lanes 1-10) of orcinol/$H_2SO_4$ stained (Sphingolipid Biochemistry, Plenum Press, S. Hakomori, (1983)) monosialoganglioside fractions from A431 cells separated by chromatography on a DEAE-Sephadex A25 column followed by HPLC on a porous silica gel column through latrobeads 6RS8010. The de-N-acetyl-GM3 containing fractions are indicated by an asterisk at the bottom. Lanes a and b are standards.

Figure 9, panel B shows the separation pattern (lanes 1-10) of immunostained (with monoclonal IgG3 antibody DH5) monoganglioside fractions from A431 cells separated as described for panel A. The de-N-acetyl-GM3 containing fractions are indicated by arrows. Lane c is a standard consisting of de-N-acetyl-GM3 and lane d is a standard consisting of GM1, GM2 and GM3.

Figure 9, panel C shows N-[$^{14}$C]-acetylated de-N-acetyl-GM3 fraction separation on HPTLC and followed by autoradiography. The position of N-[$^{14}$C]-acetylated GM3 is indicated by the arrows. Lane 1 is an N-[$^{14}$C]-acetylated de-N-acetyl-GM3 standard and lane 2 is the N-[$^{14}$C]-acetylated de-N-GM3 fraction from A431 cells.

The results in Figure 9 clearly indicate that A431 cells contain de-N-acetyl-GM3.

Figure 10 shows TLC staining of the monosialoganglioside fraction of various cell lines and tissues. Panel A shows resorcinol/HCl staining (Sphingolipid Biochemistry, Plenum Press, S. Hakomori, (1983)) and panel B shows immunostaining with the monoclonal antibody DH5.

Lane 1 is a standard with GM3, GM1, de-N-acetyl-GM3 and de-N-acetyl-GM1 (GM1 containing neuraminic acid). Lanes 2, 3, 4, 5 and 6 are fractions from B16 melanoma tumor grown in vivo, B16 cells cultured in vitro, Swiss 3T3 cells cultured in vitro, rat liver and rat brain, respectively.

From these results it is seen that B16 melanoma cells that were grown in mice as an in vivo tumor or cultured in vitro contain de-N-acetyl-GM3, whereas normal rat brain and normal 3T3 cells do not contain de-N-acetyl-GM3, and normal rat liver only contains a small amount, in relative terms, of de-N-acetyl-GM3.

Further, a significant quantity of de-N-acetyl-GM3 was found in human colonic cancer cells when treated according to the above methods.

In the above experiments, any possibility for artificial production of de-N-acetyl GM3 was eliminated by the absence of de-N-acetyl GM3 when [$^3$H]-palmetoyl GM3 was processed by the same procedure for preparation of the monosialoganglioside fraction.

## EXAMPLE II

### EFFECT OF DE-N-ACETYL-GM3 ON TYROSINE KINASE ACTIVITY OF EGF RECEPTOR

Membrane preparation and receptor kinase activity

The cell membrane fraction of A431 cells cultured as described in Example I was prepared according to the method described previously (Hanai, N. et al (1987) Biochem. Biophys. Res. Commun. 147, 127-134). Briefly, confluent cell cultures in 150 mm plastic dishes were scraped, pelleted in PBS (800 x g), and resuspended in 7 ml of 20 mM HEPES buffer (pH 7.4), 1 mM EGTA, 0.5 mM phenylmethylsulfonyl fluoride in 250 mM sucrose, homogenized in a Dounce homogenizer with a tight-fitting pestle (Weaton Scientific, Millerville, NJ), and centrifuged at 3000 x g for 10 minutes. The supernatant fraction was centrifuged at 100,000 x g for 1 hour, and the pellet was suspended in 300 μl of 200 mM HEPES buffer (pH 7.4), aliquoted, and frozen in a liquid nitrogen tank. The phosphorylation of membrane proteins, particularly EGF receptor, was performed as previously described (Bremer, E., et al (1986) J. Biol. Chem. 261, 2434-2440) with a few modifications. Briefly, cell membranes were incubated in the buffer (20 mM HEPES, pH 7.4, 1 mM MnCl2, 10 μM ZnCl2, 30 μM NaCO3) including 0.33 μM EGF (receptor grade; Collaborative Research, Waltham, MA) plus 1.5 μM carrier BSA and various concentrations of Triton X-100 (high purity detergent, Pierce, IL) in the presence or absence of gangliosides for 10 minutes at 25°C. The reaction was started by the addition of 1.0 μM or 10 μM [gamma-$^{32}$P]ATP (10 μCi) for 10 min at 0°C. The total reaction volume was 50 μl, and the amount of membrane protein was 25 μg. The reactions were terminated by addition of 50 μl of Laemmli's sample buffer (Laemmli, U.K. (1970) Nature 227, 680-685). Aliquots of the incubation mixture were subjected to 8% SDS-polyacrylamide gel electrophoresis. The gel was washed with 1 M NaOH for 15 min at 25°C, then treated with 1 M NaOH for 1 hour at 40°C to reduce serine or threonine O-phosphate (Cheng, Y.S.E. and Chen, B. (1981) Proc. Natl. Acad. Sci. USA 78, 2388-2392) and dried, followed by visualization by autoradiography. The region containing the EGF receptor (170 Kd band) was excised from the gel, and the $^{32}$P activity was determined by a liquid scintillation counter. Amino acid phosphate analysis was performed on the EGF receptor fraction excised from the gel according to the method previously described (Cooper, J.A. et al (1983) Meth. Enzymol. 99, 387-402).

The results are shown in Figures 11, 12 and 13.

Figure 11 shows the dependence of EGF receptor kinase activity of A431 cell membranes on Triton X-100 concentration. The open circles represent control samples wherein no ganglioside was added to the assay; the closed triangles represent samples wherein GM3 was added; and the closed circles represent samples wherein de-N-acetyl-GM3 was added.

The results show that EGF-dependent receptor kinase activity was strongly enhanced by addition of de-N-acetyl-GM3 with or without Triton X-100, although the receptor kinase activity is closely correlated with the concentration of this detergent in in vitro assay. EGF-dependent kinase activity was enhanced in control cells at very low detergent concentration (0.025%). In the presence of de-N-acetyl-GM3, the receptor kinase activity was several-fold higher at this low detergent concentration, and was always 2-4 fold higher regardless of detergent concentration in the assay system. When Triton X-100 concentration was lower than 0.1%, GM3 inhibited the kinase activity strongly.

Figure 12 shows the effect of de-N-acetyl-GM3 on in vitro phosphorylation of EGF receptor depending on ganglioside concentration keeping the EGF concentration constant at 0.33 μM (Panel A) and EGF concentration keeping the de-N-acetyl-GM3 concentration constant at 500 μM (Panel B). In all cases, the Triton X-100 concentration was 0.025%. The closed circles represent de-N-acetyl-GM3; the open circles represent a control without ganglioside addition; and the (X) represents no EGF.

The results indicate that the promoting effect of de-N-acetyl-GM3 on EGF-dependent receptor kinase activity increases when the concentration of de-N-acetyl-GM3 in A431 cell membranes is increased (Figure 12, panel A). The enhancing effect of de-N-acetyl-GM3 on EGF receptor kinase activity was observed even without addition of EGF, although it was more obvious when EGF concentration was increased up to 0.1-0.2 μM (Figure 12, panel B).

Figure 13 shows the phospho-amino acid pattern in a hydrolysate of the EGF receptor band taken from the gel region containing the EGF receptor. The gel region was excised and hydrolyzed with 5.7 M HCl at 110°C for 1 hour. At the end of the hydrolysis, the mixture was lyophilized and spotted onto a 20 x 20-cm cellulose thin-layer plate. Two-dimensional thin-layer electrophoresis was carried out at pH 1.9 and at pH 3.5.

Panel A shows the results where the sample contained only EGF and Panel B shows the results where the sample contained EGF and de-N-acetyl-GM$_3$

The results show that when phosphoamino acid analysis was performed on the hydrolysate of the EGF receptor in the presence or absence of de-N-acetyl-GM$_3$, enhanced phosphorylation was found corresponding only to tyrosine phosphate, but not serine or threonine phosphate.

<div align="center">EXAMPLE III</div>

<div align="center">EFFECT OF DE-N-ACETYL-GM$_3$ AND GM$_3$ ON CELL GROWTH</div>

A431 cells, Swiss 3T3 cells, and B16 cells were seeded into 96-well plates in serum-free medium as described in Example I at a density of 0.4 x 10$^4$ cells/well. On the third day of culture, 10 μM de-N-acetyl-GM$_3$ was added to A431 cells; 50 μM de-N-acetyl-GM$_3$ or 50 μM GM$_3$ was added to Swiss 3T3 cells; 50 μM de-N-acetyl-GM$_3$ or 50 μM GM$_3$ was added to B16 cells. For a control, no ganglioside was added.

Each day the number of cells was counted and the average of 4 determinations plus or minus standard error was taken as the number of cells.

Purified gangliosides were added to the cell culture medium as follows. Ganglioside in chloroform:methanol solution was transferred to a glass test tube, evaporated under a nitrogen stream, dissolved in culture medium at the indicated concentration, sonicated, and passed through a 0.2μ sterilizing filter.

The results are shown in Figure 14 where Panel A represents A431 cells, panel B represents Swiss 3T3 cells and panel C represents B16 cells. Closed circles represent de-N-acetyl-GM$_3$ addition; closed triangles represent GM$_3$ addition; and open circles represent no ganglioside addition.

The results show that A431 cell growth was significantly enhanced when cells were cultured in medium to which de-N-acetyl-GM$_3$ was exogenously added (Figure 14, panel A) as compared with cells cultured in medium only. A growth promoting effect by de-N-acetyl-GM$_3$ was most pronounced with 3T3 cells, in which GM$_3$ was strongly growth-inhibitory (Figure 14, panel B). Growth of B16 melanoma cells was strongly inhibited by GM$_3$ but not affected by de-N-acetyl-GM$_3$ (Figure 14, panel C).

While the invention has been described in detail and with reference to specific embodiments thereof, it will be apparent to one skilled in the art that various changes and modifications can be made therein without departing from the spirit and scope thereof. In particular those skilled in the art will appreciate that references to humans may also apply to all mammals

## Claims

1. A substantially pure ganglioside containing de-N-acetyl-sialic acid.
2. The ganglioside of Claim 1, which comprises de-N-acetyl-GM$_3$.
3. The ganglioside of Claim 1, which comprises de-N-acetyl-GM$_1$.
4. A culture medium for stimulating growth of human and animal cells comprising:
   (1) essential nutrients for cell growth, and
   (2) a cell growth stimulatory amount of one or more gangliosides containing de-N-acetyl-sialic acid.
5. The culture medium of Claim 4, wherein said one or more gangliosides containing de-N-acetyl-sialic acid comprises de-N-acetyl-GM$_3$.
6. The culture medium of Claim 4, wherein said one or more gangliosides containing de-N-acetyl-sialic acid comprises de-N-acetyl-GM$_1$.
7. The culture medium of Claim 4, wherein said cells are mammalian cells.
8. The culture medium of Claim 4, wherein said cells have insulin-dependent growth.
9. The culture medium of Claim 8, wherein said one or more gangliosides containing de-N-acetyl-sialic acid comprises sialyl 2→3 lactonorhexaosylceramide or sialyl 2→3 lactoneotetraosylceramide containing de-N-acetyl-sialic acid.
10. The culture medium of Claim 8, wherein said one or more gangliosides containing de-N-acetyl-sialic acid comprises sialyl 2→3 lactoneotetraosylceramide containing de-N-acetyl-sialic acid.
11. A method for stimulating growth of human and animal cells cultured in vitro comprising contacting said cells in culture with a cell growth stimulatory amount of one or more gangliosides containing de-N-acetyl-sialic acid.
12. The method of Claim 11, wherein said one or more gangliosides containing de-N-acetyl-sialic acid comprises de-N-acetyl-GM$_3$.
13. The method of Claim 11, wherein said one or more gangliosides containing de-N-acetyl-sialic acid comprises de-N-acetyl-GM$_1$.

<div align="center">13</div>

14. The method of Claim 11, wherein said cells are mammalian cells.

15. The method of Claim 11, wherein said cells have insulin-dependent growth.

16. The method of Claim 15, wherein said one or more gangliosides containing de-N-acetyl-sialic acid comprises sialyl 2→3 lactonorhexaosylceramide or sialyl 2→3 lactoneotetraosylceramide containing de-N-acetyl-sialic acid.

17. The method of claim 15, wherein said one or more gangliosides containing de-N-acetyl-sialic acid comprises sialyl 2→3 lactoneotetraosylceramide containing de-N-acetyl-sialic acid.

18. A medicament comprising:
(1) one or more growth stimulators selected from the group consisting of gangliosides containing de-N-acetyl-sialic acid, substances that promote synthesis of gangliosides containing de-N-acetyl-sialic acid and pharmaceutically acceptable salts thereof; and
(2) a pharmaceutically acceptable carrier, diluent or excipient.

19. The medicament of Claim 18, wherein said one or more growth stimulators comprise gangliosides containing de-N-acetyl-sialic acid or pharmaceutically acceptable salts thereof.

20. The medicament of Claim 19, wherein said gangliosides containing de-N-acetyl-sialic acid or pharmaceutically acceptable salts thereof comprise de-N-acetyl-GM$_3$ or pharmaceutically acceptable salts thereof.

21. The medicament of Claim 19, wherein said gangliosides containing de-N-acetyl-sialic acid or pharmaceutically acceptable salts thereof comprise de-N-acetyl-GM$_1$ or pharmaceutically acceptable salts thereof.

22. The medicament of Claim 18, wherein said one or more cell growth stimulators comprise substances that promote synthesis of gangliosides containing de-N-acetyl-sialic acid or pharmaceutically acceptable salts of said substances.

23. The medicament of Claim 18, wherein said substances that promote synthesis of gangliosides containing de-N-acetyl-sialic acid or pharmaceutically acceptable salts of said substances comprise N-acetylase or pharmaceutically acceptable salts thereof.

24. The medicament of claim 18, wherein said cells are mammalian cells.

25. The medicament of claim 18, wherein said cells are cells involved in wound healing.

26. The medicament of claim 18, wherein said medicament promotes wound healing.

27. The medicament of claim 18, wherein said cells have insulin-dependent growth.

28. The medicament of claim 27, wherein said one or more gangliosides containing de-N-acetyl-sialic acid comprises sialyl 2→3 lactonetetraosylceramide containing de-N-acetyl-sialic acid.

29. The medicament of claim 27, wherein said one or more gangliosides containing de-N-acetyl-sialic acid comprises sialyl 2→3 lactoneotetraosylceramide containing de-N-acetyl-sialic acid.

30. The use of one or more growth stimulators selected from the group consisting of gangliosides containing de-N-acetyl-sialic acid, substances that promotes synthesis of gangliosides containing de-N-acetyl-sialic acid, and pharmaceutically acceptable salts thereof in preparing a medicament for promoting cell growth.

31. A medicament comprising:
(1). one or more growth inhibitors that block synthesis of gangliosides containing de-N-acetyl-sialic acid or pharmaceutically acceptable salts thereof; and
(2). a pharmaceutically acceptable carrier, diluent or excipient.

32. The medicament of claim 31, wherein said one or more growth inhibitors that block synthesis of gangliosides containing de-N-acetyl-sialic acid or pharmaceutically acceptable salts of said substances comprise anti-N-deacetylase or pharmaceutically acceptable salts thereof.

33. The medicament of claim 31, wherein said one or more growth inhibitors are gangliosides or lyso gangliosides comprising N-modified sialic acid.

34. The medicament of claim 33, wherein said gangliosides or lyso form gangliosides comprising N-modified sialic acid comprises gangliosides or lyso form gangliosides containing N-carbamyl acetyl sialic acid, N-propyl sialic acid, N-dichloroacetyl sialic acid, N-trichloroacetyl sialic acid or N-trifluoroacetyl sialic acid.

35. The medicament of claim 31, wherein said cells are mammalian cells.

36. The medicament of claim 31, wherein said cells are malignant or non-malignant tumor cells.

37. The medicament of claim 31, wherein said medicament prevents or slows tumor growth.

38. The medicament of claim 31, wherein said medicament prevents or slows metastasis of tumors.

39. The medicament of claim 31, wherein said cells have insulin-dependent growth.

40. The use of one or more growth inhibitors that block synthesis of gangliosides containg de-N-acetyl-sialic acid or pharmaceutically acceptable salts thereof in preparing a medicament for inhibiting cell growth.